(19) Europäisches Patentamt
European Patent Office
Office européen des brevets

(11) Publication number: **0 294 789 B1**

(12) **EUROPEAN PATENT SPECIFICATION**

(45) Date of publication of patent specification: **12.10.94**

(51) Int. Cl.5: **C07D 499/68**, C07D 417/12, //C07D277/72

(21) Application number: **88109147.4**

(22) Date of filing: **08.06.88**

The file contains technical information submitted after the application was filed and not included in this specification

# (54) Process for preparing 6-/D(-)-Alpha-(4-ethyl-2,3-dioxo-1-piperazine-carboxamido)-phenylacetamido/pennicillanic acid.

(30) Priority: **11.06.87 YU 1089/87**

(43) Date of publication of application: **14.12.88 Bulletin 88/50**

(45) Publication of the grant of the patent: **12.10.94 Bulletin 94/41**

(84) Designated Contracting States: **AT CH DE ES FR GB GR IT LI SE**

(56) References cited:
**EP-A- 0 037 380**
**EP-A- 0 096 297**
**EP-A- 0 115 770**
**EP-A- 0 187 209**
**US-A- 4 316 024**

**CHEMICAL ABSTRACTS, vol. 106, no. 3, 19th January 1987, page 577, no. 18250y, Columbus, Ohio, US;**

(73) Proprietor: **KRKA, tovarna zdravil, n.sol.o**
**Cesta herojev 45**
**YU-68000 Novo Mesto (YU)**

(72) Inventor: **Zupet, Pavel**
**Mackovec 12a**
**YU-68000 Novo Mesto (YU)**
Inventor: **Vitezic, Natalija**
**Ragovska 6a**
**YU-68000 Novo Mesto (YU)**

(74) Representative: **Müller-Boré & Partner Patentanwälte**
**Postfach 26 02 47**
**D-80059 München (DE)**

EP 0 294 789 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Technical Field

The present invention relates to the field of organic chemistry synthesis and comprises a novel economical process for preparing 6-[D(-)-alpha-(4-ethyl-2,3-dioxo-1-piperazinecarboxamido)-phenylacetamido]penicillanic acid (piperacillin) as well as pharmaceutically acceptable alkali metal salts thereof of the formula (I)

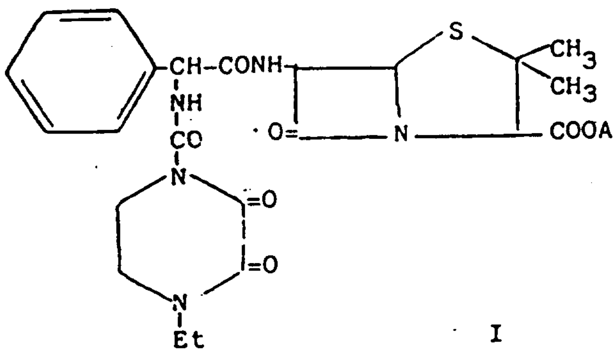

I

wherein A represents a hydrogen atom, a sodium atom or a potassium atom.

The compound of the formula (I) is a semisynthetic antibiotic which is very active against Gram-negative bacteria Escherichia coli, Klebsiella spp., Salmonella spp., and Gram-positive bacteria Staphylococcus aureus, Streptococcus spp., as well as against anaerobic bacteria Bacteroides fragilis, Peptococcus spp.

### Technical Problem

There was a need to find an economical process for the synthesis of 6-[D(-)-alpha-(4-ethyl-2,3-dioxo-1-piperazinecarboxamido)-phenylacetamido]penicillanic acid from a novel intermediate - a derivative of 4-ethyl-2,3-dioxo-1-piperazine, which would be stable and could be employed in the synthsis also after a prolonged period of transport and storage. It is, namely, well known that the basic intermediate 4-ethyl-2,3-dioxo-1-piperazine-4-carbonylchloride, which is usually employed in the synthesis of piperacillin, is unstable and must be used for further reaction immediately after preparation. The synthesis of 4-ethyl-2,3-dioxo-1-piperazine-4-carbonylchloride is carried out only by the use of phosgene, which requires special equipment and security measures. Therefore in Europe there exists only a small number of manufacturers that are able to carry out said synthesis on a larger scale. In an additional step they could prepare the desired stable derivative, useful as a novel intermediate for the synthesis according to the invention, from said unstable derivative.

### Prior Art

The synthesis of the compound (I) is described in Yakugaku Zasshi 97(9), 980-986 (1977), and in DE 2519400 C3. US-A-4 316 024 (A) discloses the production of cephalosporins carrying side-groups very similar in structure to that of piperacillin by the use of an active thioester of the piperazinyl moiety. Active thioesters containing the benzothiazole unit and being used in the field of $\beta$-lactam synthesis are known from EP-A-0 096 297 (B), EP-A-0 037 380 (C), EP-A-0 187 209 (D) and EP-A-0 115 770 (E).

In the known process the compound of the formula (I) is prepared by the condensation of alkali salts of D(-)-alpha-amino-benzylpenicillin (ampicillin) and 4-ethyl-2,3-dioxo-1-piperazinecarbonylchloride in an aqueous medium. In anhdyrous media condensation takes place between the silylated form of D(-)-alpha-aminobenzylpenicillin and 4-ethyl-2,3-dioxo-1-piperazinecarbonylchloride.

In all known cases the unstable 4-ethyl-2,3-dioxo-1-piperazine-carbonylchloride is used as a reactant.

The Inventive Solution

The process for preparing a compund of the formula (I) is carried out in such a way that an active thioester of 4-ethyl-2,3-dioxo-1-piperazine-carboxylic acid of the formula (II)

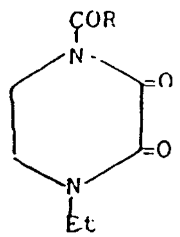

II

wherein R represents a 2-mercaptobenzothiazole radical, is condensed with a silylated form of 6-[D(-)-alpha-aminophenylacetamido]penicillanic acid (ampicillin) of the formula (III)

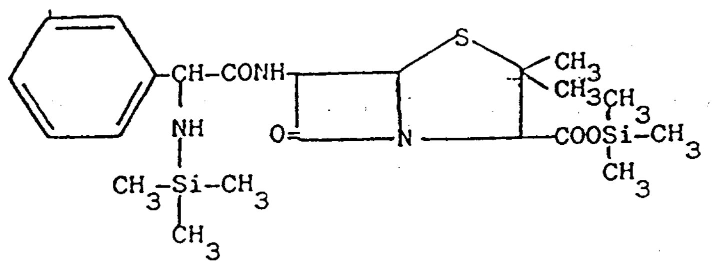

III

The condensation reaction is carried out in inert organic solvents, preferably in methylene chloride, and in the temperature range from 0°C to reflux temperature of a solvent. The most suitable reaction temperature is 20°C ± 5°C. The condensation is rapid and is completed already after 3 hours. After the completion of the reaction the silylated piperacillin is hydrolysed with an addition of alkaline demineralized water (pH = 6.8 to 7.3) or demineralized water and, after the separation of the organic solvent, the end product is isolated from water or an aqueous-alkaline medium in the form of an acid by addition of diluted HCl at pH = 2.1 to 2.3. The product is obtained in high yield and good quality. The obtained acid is converted to sodium salt with sodium 2-ethylhexanoate in acetone. Secondary products, such as 2-mercaptobenzothiazole, can be recovered from the organic solvent and reused in the synthesis of thioesters of the formula (II).

Ampicillin, from which the silylated form of the formula (III) is prepared, is commercially available.

The compound of the formula (II) is a novel compound and it also represents one of the objects of the present invention.

It can be obtained from freshly prepared 4-ethyl-2,3-dioxo-1-piperazine-carbonylchloride of the formula (IV)

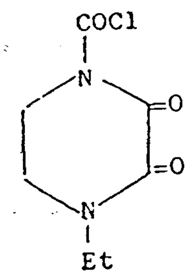

IV

and 2-mercaptobenzothiazole. The active thioester of the formula (II) can be prepared in inert organic solvents, preferably in methylene chloride, in the presence of a base, preferably triethylamine, and at a temperature from 0°C to the reflux temperature of the solvent, preferably at room temperature.

The thioester of the formula (II) is a solid, stable, non-aggressive compound, very suitable for transport and storage, which makes it advantageous over the sensitive and aggressive 4-ethyl-2,3-dioxo-1-piperazinecarbonylchloride,which decomposes rapidly at elevated temperature and humidity and must therefore be stored and transported in the cool.

The novel thioester of the formula (II) is a reactive compound, which at room temperature smoothly reacts with silylated ampicillin in an organic solvent in dry conditions, preferably in methylene chloride, and in the reaction the compound (I) piperacillin is formed in high yield and good quality (negligible presence of decomposition products - not more than 1 %).

The condensation reaction between compounds (II) and (III) is carried out at room temperature and so is the isolation of the final piperacillin, thus avoiding energy losses. The total reaction is completed in a shorter time.

After the reaction secondary products as 2-mercapto -benzothiazole can be regenerated from the organic layer (e.g. methylene chloride layer) after distillation.

The process is illustrated in detail by the following Examples, which are in no way a limitation thereof.

## Example 1

6-[D(-)-alpha-(4-ethyl-2,3-dioxo-1-piperazinecarboxamido)-phenylacetamido]penicillanic acid monohydrate

6-[D(-)-alpha-aminophenylacetamido]penicillanic acid (anhydrous ampicillin; 35 g; 0.1 mole) is suspended in anhydrous methylene chloride (500 ml) and N,0 - bis (trimethylsilyl)acetamide (35 ml; 0.14 mole) is added under stirring at room temperature. The reaction mixture is stirred for one hour at room temperature whereby ampicillin is silylated and is soluble in methylene chloride. Then S-2-benzothiazolyl-4-ethyl-2,3-dioxo-1-piperazinethio-carboxylate (33.5 g; 0.1 mole) is added and the stirring is continued for 3 hours at 22 to 24°C. After the completed reaction a solution of $KHCO_3$ (10 g) in demineralized water (500 ml) is added to the reaction mixture and it is stirred for 0.5 hours (pH is 6.8). After the completed hydrolysis the methylene chloride layer is separated from the aqueous layer and washed with demineralized water (100 ml). Aqueous layers are combined, the remaining methylene chloride is evaporated i.v. and filtered. A 10% aqueous HCl solution is added under vigorous stirring at room temperature to the clear filtrate till the pH is 2.1 - 2.2.

The stirring is reduced and continued for another 3 hours. The product is filtered off, washed with demineralized water and dried at 35°C in a vacuum drier to a humidity of 4% according to Karl Fischer (K.F.).

6-[D(-)-alpha-(4-ethyl-2,3-dioxo-1-piperazinecarboxamido)-phenylacetamido]penicillanic acid monohydrate (4.8 g; yield 90 %) is obtained.

The structure of the compound was confirmed with IR and NMR spectra.

Optical rotation $(\alpha)_D^{20}$ = 170° dry substance

Humidity (K.F.) = 3.35 %

Contents: 994 µg/mg of dry substance (microbiologically) 99,0 % of dry substance (HPLC)

## Example 2

6-[D(-)-alpha-(4-ethyl-2,3-dioxo-1-piperazinecarboxamido)-phenylacetamido]penicillanic acid sodium salt

6-[D(-)-alpha -aminophenylacetamido]penicillanic acid (ampicillin; 35 g; 0.1 mole) is suspended in dry methylene chloride (350 ml). The mixture is cooled to 15°C and triethylamine (29 ml; 0.2 mole) is slowly added drop by drop, it is waited for 20 minutes for the dissolution of all ampicillin and then the addition of trimethylchlorosilane (28.3 ml; 0.22 mole) begins. It is stirred for 1 hour at room temperature and S-2-benzothiazolyl-4-ethyl-2,3-dioxo-1-piperazinethio-carboxylate (33.5 g; 0.1 mole) is added. The reaction mixture is stirred for 3 hours at room temperature, demineralized water (450 ml) is added and the pH corrected with $KHCO_3$ to 7-7.5. Layers are separated and the organic layer is washed with demineralized water (100 ml). The aqueous layers are combined and the remaining methylene chloride is distilled off i.v. Diluted hydrochloric acid (1:5) is added under stirring to the clear aqueous layer to pH 2.1-2.2.

The obtained product is filtered, washed with demineralized water and dried to humidity 4 % (K.F.).

The obtained product (44 g; 0.085 mole) is suspended in acetone (386 ml) at room temperature and under stirring a solution of sodium 2-ethylhexanoate (14.1 g; 0.085 mole) in acetone (193 ml) is added drop by drop. It is stirred for another 0.5 hour, the obtained product is filtered, washed with acetone and dried in a vacuum drier at 35°C.

6-[D(-)-alpha-(4-ethyl-2,3-dioxo-1-piperazinecarboxamido)-phenylacetamido]penicillanic acid sodium salt (42g; yield 77 %) is obtained.

The structure of the compound was confirmed with IR and NMR spectra.

Humidity (K.F.): 1.69 %

Optical rotation: $(\alpha)_D^{20}$ = + 182,64° dry substance

Contents: 956 µg/mg of dry substance (microbiologically) 98.26 % of dry substance (HPLC)

Example 3

6-[D(-)-alpha-(4-ethyl-2,3-dioxo-1-piperazinecarboxamido)-phenylacetamido]penicillanic acid monohydrate

6-[D(-)-alpha-aminophenylacetamido]penicillanic acid (35 g; 0.1 mole) is suspended in anhydrous methylene chloride (500 mi) and under stirring at room temperature N,O-bis(trimethylsilyl)acetamide (35 ml; 0.14 mole) is added. The reaction mixture is stirred for 1 hour at room temperature so that ampicillin is silylated and becomes soluble in methylene chloride. Then S-2-benzothiazolyl-4-ethyl-2,3-dioxo-1-piperazinethio-carboxylate (33.5 g; 0.1 mole) is added and it is stirred for 3 hours at a temperature of 22 to 24°C. The reaction mixture is poured into demineralized water (500 ml) under vigorous stirring and the pH is adjusted to 1.5 with diluted hydrochloric acid (1 : 5). It is stirred for 30 minutes at room temperature and the layers are separated. The aqueous layer is washed with methylene chloride (200 ml). The organic layers are combined, under stirring demineralized water (100 ml) is added and the pH is adjusted to 7.1 - 7.3 with diluted ammonia. The layers are again separated and the methylene chloride layer washed with demineralized water (600 ml). The aqueous layers are combined and the remaining methylene chloride is evaporated i.v. Methanol (180 ml) is added to the solution, it is heated to 35°C and under vigorous stirring it is acidified with diluted HCl to pH 2.1 - 2.4. The reaction mixture is cooled to 5 to 10°C and stirred for 2 hours.

The product is filtered, washed with demineralized water and dried in an air-drier at 35°C to humidity 4% (K.F.).

6-[D(-)-alpha-(4-ethyl-2,3-dioxo-1-piperazinecarboxamido)-phenylacetamido]penicillanic acid monohydrate (44 g; yield 85 %) is obtained.

The structure of the compound was confirmed with IR and NMR spectra.

Humidity (K.F.): 3.84 %

Optical rotation: $(\alpha)_D^{20}$ = + 170,5° dry substance

Contents: 992 µg/mg of dry substance (microbiologically) 98.3 % of dry substance (HPLC).

Example 4

2-mercaptobenzothiazole

To methylene chloride layer (500 ml), which remains after hydrolysis and extraction of piperacillin, 10 % hydrochloric acid (200 ml; pH = 0.5-1.0) is added under stirring at room temperature. The mixture is stirred for 4 hours and the obtained precipitate is filtered.

2-mercaptobenzothiazole (9 g; yield 54 %) with m.p. 174-178°C is obtained.

Example 5

S-2-benzothiazolyl-4-ethyl-2,3-dioxo-1-piperazinethiocarboxylate

2-mercaptobenzothiazole (16.7g; 0.1 mole) is suspended in methylene chloride (500 ml) and triethylamine (14 ml; 0.1 mole) is added at room temperature under stirring. After the solution is formed 4-ethyl-2,3-dioxo-1-piperazine-carbonylchloride (20.4 g; 0.1 mole) is added under stirring. The reaction mixture is stirred for another 4 hours at room temperature, filtered and the obtained product is washed with acetone (50 ml). After drying in an air-drier S-2-benzothiazolyl-4-ethyl-2,3-dioxo-1-piperazinethiocarboxylate (30 g; yield 93 %) with m.p. 217-220°C is obtained.

The structure of the product was confirmed with IR and NMR spectra.

## Claims

**1.** Process for preparing 6-[D(-)-alpha-(4-ethyl-2,3-dioxo-1-piperazinecarboxamido)-phenylacetamido]-penicillanic acid as well as pharmaceutically acceptable alkali metal salts thereof of the formula (I)

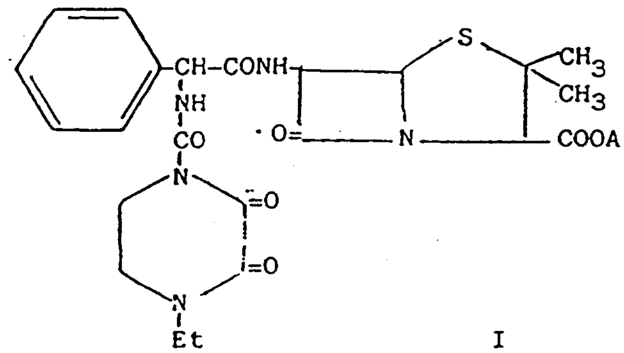

I

wherein A represents a hydrogen atom, a sodium atom or a potassium atom, characterized in that active thioesters of 4-ethyl-2,3-dioxo-1-piperazinecarboxylic acid of the formula (II)

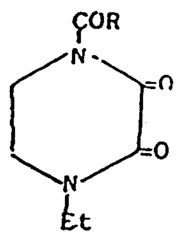

II

wherein R represents a 2-mercaptobenzothiazole radical, is condensed with a silylated form of 6-[D(-)-alpha-aminophenylacetamido]penicillanic acid of the formula (III)

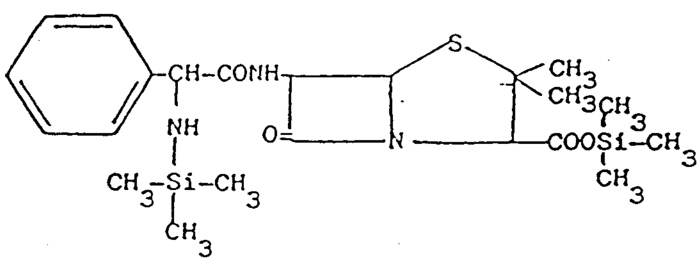

III

and then hydrolysed at a pH of 6.8 to 7.3.

**2.** Process according to claim 1, characterized in that the condensation is carried out in inert organic solvents and in the temperature range from 0°C to reflux temperature of the solvent.

**3.** Process according to claim 2, characterized in that the temperature is room temperature.

**4.** Active thioester of the formula (II)

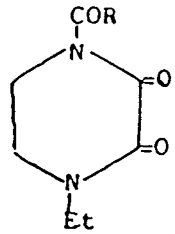

II

wherein R represents a 2-mercaptobenzothiazole radical.

**5.** Process for preparing an active thioester of the formula (II)

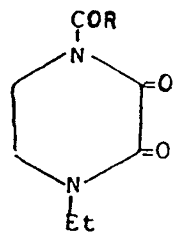

IJ

wherein R represents a 2-mercaptobenzothiazole radical, characterized in that freshly prepared 4-ethyl-2,3-dioxo-1-piperazinecarbonylchloride of the formula (IV)

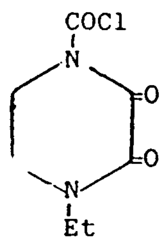

IV

is reacted with 2-mercaptobenzothiazole.

**6.** Process according to claim 5, characterized in that the reaction is carried out in inert organic solvents, in the presence of a base, and at a temperature from 0°C to reflux temperature of the solvent.

**7.** Process according to claims 5 and 6, characterized in that the temperature is room temperature.

**8.** Process according to claims 5 and 6, characterized in that the inert organic solvent is methylene chloride.

**9.** Process according to claims 5 and 6, characterized in that the base is triethylamine.

**10.** Process according to claim 1, characterized in that after completed reaction, hydrolysis and extraction of the compound of the formula (I) the organic phase is treated and there is obtained 2-mercaptobenzothiazole, which is employed in synthesis of the compound of the formula (II).

## Patentansprüche

1. Verfahren zur Herstellung von 6-[D(-)-α-(4-Äthyl-2,3-dioxo-1-piperazincarboxamido)-phenylacetamido]-penicillansäure sowie deren pharmazeutisch annehmbaren Alkalimetallsalzen der Formel (I)

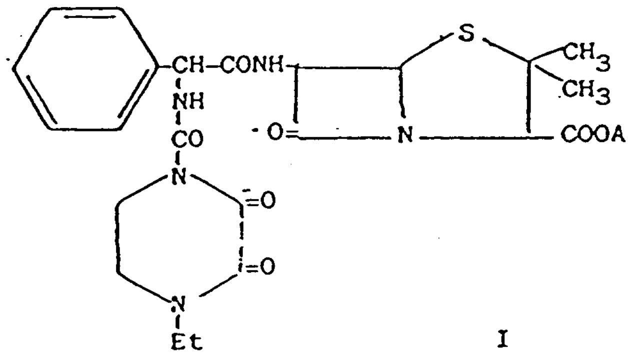

I

worin A ein Wasserstoffatom, ein Natriumatom oder ein Kaliumatom darstellt, dadurch gekennzeichnet, dass der Aktivthioester der 4-Äthyl-2,3-dioxo-1-piperazincarbonsäure der Formel (II)

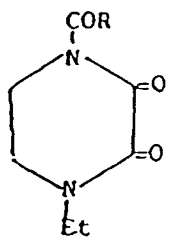

II

worin R einen 2-Mercaptobenzothiazolrest darstellt, mit 6-[D(-)-α-aminophenylacetamido]penicillansäure in der silylierten Form der Formel (III)

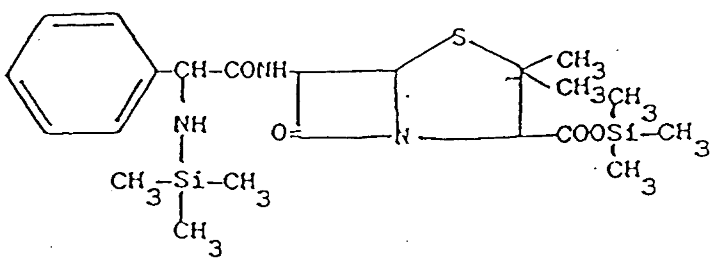

III

kondensiert wird und anschliessend bei einem pH-Wert von 6,8 bis 7,3 hydrolysiert wird.

2. Verfahren nach Anspruch 1, dadurch gekennzeichnet, dass die Kondensation in einem inerten organischen Lösungsmittel und in einem Temperaturbereich von 0°C bis Rückflusstemperatur des Lösungsmittels durchgeführt wird.

3. Verfahren nach Anspruch 2, dadurch gekennzeichnet, dass die Temperatur Raumtemperatur ist.

4. Aktivthioester der Formel (II)

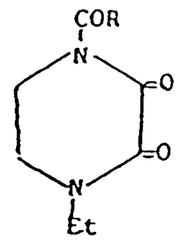

II

worin R einen 2-Mercaptobenzothiazolrest darstellt.

5. Verfahren zur Herstellung des Aktivthioesters der Formel (II)

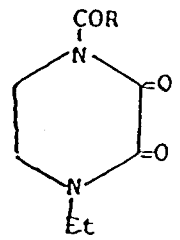

II

worin R einen 2-Mercaptobenzothiazolrest darstellt, dadurch gekennzeichnet, dass frisch hergestelltes 4-Äthyl-2,3-dioxo-1-piperazincarbonylchlorid der Formel (IV)

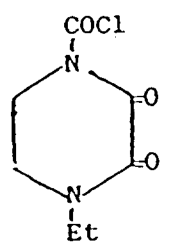

IV

mit 2-Mercaptobenzothiazol umgesetzt wird.

6. Verfahren nach Anspruch 5, dadurch gekennzeichnet, dass die Umsetzung in einem inerten organischen Lösungsmittel in Gegenwart einer Base und bei einer Temperatur von 0°C bis Rückflusstemperatur des Lösungsmittels durchgeführt wird.

7. Verfahren nach Ansprüchen 5 und 6, dadurch gekennzeichnet, dass die Temperatur Raumtemperatur ist.

8. Verfahren nach Ansprüchen 5 und 6, dadurch gekennzeichnet, dass das inerte organische Lösungsmittel Methylenchlorid ist.

9. Verfahren nach Ansprüchen 5 und 6, dadurch gekennzeichnet, dass die Base Triethylamin ist.

10. Verfahren nach Anspruch 1, dadurch gekennzeichnet, dass nach der abgeschlossenen Umsetzung, Hydrolyse und Extraktion der Verbindung der Formel (I) die organische Phase bearbeitet wird und 2-Mercaptobenzothiazol erhalten wird, das in der Synthese der Verbindung der Formel (II) weiterverwendet wird.

## Revendications

1. Procédé de préparation de l'acide 6-[D(-)-alpha-(4-éthyl-2,3-dioxo-1-pipérazinecarboxamido)-phénylacétamido]pénicillanique, ainsi que des sels de métal alcalin pharmaceutiquement acceptables de celui-ci, répondant à la formule (I) :

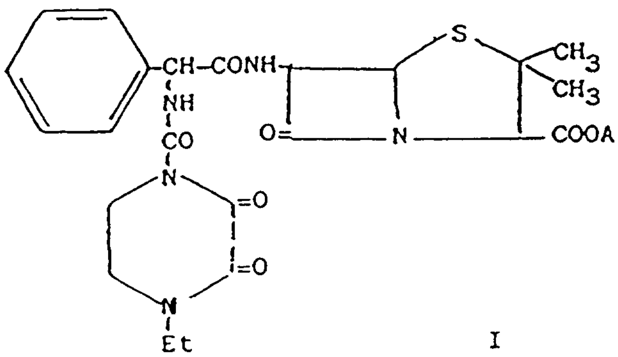

I

où A représente un atome d'hydrogène, un atome de sodium ou un atome de potassium,
caractérisé en ce que l'on condense des thioesters actifs de l'acide 4-éthyl-2,3-dioxo-1-pipérazinecarboxylique de formule (II) :

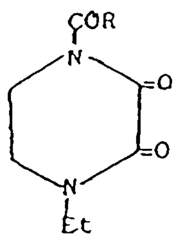

II

où R représente un radical 2-mercaptobenzothiazole, avec une forme silylée de l'acide 6-[D(-)-alpha-aminophénylacétamido]pénicillanique de formule (III) :

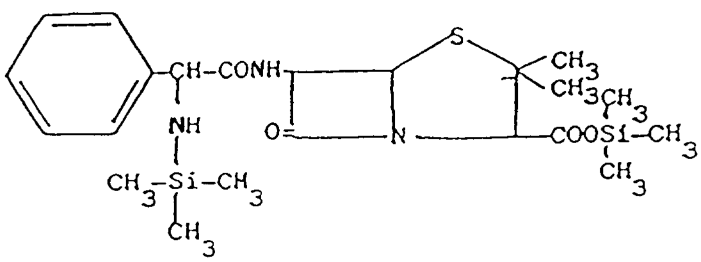

III

et ensuite, on hydrolyse le condensat à pH 6,8 à 7,3.

2. Procédé selon la revendication 1, caractérisé en ce qu'on effectue la condensation dans des solvants organiques inertes et à une température comprise entre 0°Cet la température de reflux du solvant.

3. Procédé selon la revendication 2, caractérisé en ce que la température est la température ambiante.

**4.** Thioester actif répondant à la formule (II) :

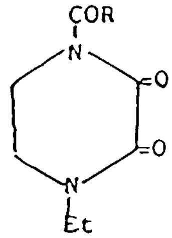

II

où R représente un radical 2-mercaptobenzothiazole.

**5.** Procédé de préparation d'un thioester actif répondant à la formule (II) :

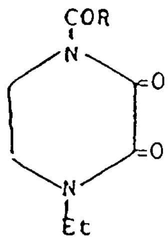

II

où R représente un radical 2-mercaptobenzothiazole, caractérisé en ce qu'on fait réagir du chlorure de 4-éthyl-2,3-dioxo-1-pipérazinecarbonyle fraîchement préparé, répondant à la formule (IV) :

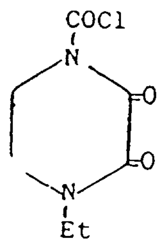

IV

avec du 2-mercaptobenzothiazole.

**6.** Procédé selon la revendication 5, caractérisé en ce qu'on effectue la réaction dans des solvants organiques inertes, en présence d'une base et à une température comprise entre 0°C et la température de reflux du solvant.

**7.** Procédé selon les revendications 5 et 6, caractérisé en ce que la température est la température ambiante.

**8.** Procédé selon les revendications 5 et 6, caractérisé en ce que le solvant organique inerte est le chlorure de méthylène.

**9.** Procédé selon les revendications 5 et 6, caractérisé en ce que la base est la triéthylamine.

**10.** Procédé selon la revendication 1, caractérisé en ce que, lorsque la réaction, l'hydrolyse et l'extraction du composé de formule I sont terminées, on traite la phase organique et on obtient du 2-mercaptobenzothiazole que l'on emploie dans la synthèse du composé de formule (II).